# EUROPEAN PATENT APPLICATION

(11) **EP 2 554 200 A1**
(43) Date of publication of application: **06.02.2013**
(21) Application number: 11765461.6
(22) Date of filing: 25.03.2011
(51) Int. Cl.: A61M 5/24, A61M 5/28, A61M 5/32

(54) **PREFILLED SYRINGE**

(30) Priority: 31.03.2010 JP 2010082578
(71) Applicant: Terumo Kabushiki Kaisha, Tokyo 151-0072 (JP)
(72) Inventor: OGAWA Junichi, Nakakoma-gun Yamanashi 409-3853 (JP); TACHIKAWA Kouichi, Nakakoma-gun Yamanashi 409-3853 (JP); OTSU Shinnosuke, Nakakoma-gun Yamanashi 409-3853 (JP)
(74) Representative: TBK
(86) International application number: PCT/JP2011/057466
(87) International publication number: WO 2011/125560

(57) **Abstract**

A prefilled syringe is configured so that, in order that a liquid medicine injected and administered into the upper layer part of skin is prevented from leaking out of the living body, a pressing section can be held in a position reached when it has been completely pressed in. A prefilled syringe 1 is provided with: a double-ended needle 3 having a needlepoint 9 which can be inserted into a living body; a needle hub 4 for holding the double-ended needle 3; an outer tube 2; a pressing section 22; a liquid medicine storage section 6 for storing a liquid medicine M; a holding section 8; and a sealing body 23. The liquid medicine storage section 6 is disposed within a tube hole 2a of the outer tube 2. The pressing section 22 operates the liquid medicine storage section 6 so that the liquid medicine storage section 6 can move in the axial direction of the tube hole 2a of the outer tube 2. In a pressed state in which the liquid medicine storage section 6 and the pressing section 22 have been moved toward the needle hub 4 side until the liquid medicine M stored within the liquid medicine storage section 6 has been discharged, the holding section 8 holds the pressed state of the pressing section 22.

## Description

The present invention relates to a prefilled syringe wherein a liquid medicine is preliminarily stored in a syringe.

### BACKGROUND ART

In recent years, an increasing number of prefilled syringes wherein a liquid medicine is preliminarily stored in a syringe have come to be used (Patent Document 1). In such a prefilled syringe, it is unnecessary to suck a liquid medicine from a vial into a syringe at the time of liquid medicine administration. Therefore, the time required for administration can be shortened, and the liquid medicine can be restrained from being wasted.

Furthermore, infection of avian influenza to humans has been reported, and there is anxiety about vast damage due to the outbreak (pandemic) of human-to-human infection of the avian influenza. In view of this, saving of a pre-pandemic vaccine which is highly possibly effective in controlling the avian influenza is now conducted worldwide. Besides, in order to administer the pre-pandemic vaccine to many humans, investigations for increasing the production output of the vaccine are underway.

A skin is composed of three parts, namely, the epidermis, the dermis, and the subcutaneous tissue (hypodermis). The epidermis is a layer of about 50 to 200 µm from the skin surface, and the dermis is a layer of about 1.5 to 3.5 mm following the epidermis. In general, the influenza vaccine is put to subcutaneous administration or intramuscular administration; thus, the influenza vaccine is administered into a lower layer part of the skin or a further deeper region.

On the other hand, it has been reported that by administering an influenza vaccine by adopting as a target site an upper layer part of skin where many immunocytes are present, an immune acquiring ability comparable to that by subcutaneous administration or intramuscular administration can be obtained even with a reduced dose (Non-patent Document 1). Therefore, administration of the pre-pandemic vaccine into the upper layer part of skin permits a reduction in the dose, and, accordingly, enables the pre-pandemic vaccine to be administered into a greater number of humans. Incidentally, the upper layer part of skin means the epidermis and the dermis of the skin.

As a method for administering a liquid medicine into the upper layer part of skin, there have been reported a variety of methods based on the use of a single needle, multiple needles, a patch, gas or the like. Taking administration safety, reliability and production cost into account, the method based on the use of a single needle is said to be the most suitable of the methods for administering a liquid medicine into the upper layer part of skin. As a method of administering a vaccine into the upper layer part of skin by use of a single needle, the Mantoux method has long been known. The Mantoux method is generally a method wherein a needle having a short-bevel needlepoint of 26 to 27 gauge in size is inserted into a skin by about 2 to 5 mm from an oblique direction of about 10 to 15 degrees against the skin, and a liquid medicine in an amount of about 100 µL is administered.

The administration of a liquid medicine by the Mantoux method, however, is difficult on a technical basis, and the success rate is entrusted to skilled technique of the doctor who performs the injection. In particular, a child may probably move at the time of administration, and, therefore, it is difficult to administer the influenza vaccine into a child by the Mantoux method. Accordingly, there is a demand for development of a device by which a vaccine can be simply administered into an upper layer part of skin.

Patent Document 2 describes an injection device wherein a limiter having a skin contact surface is connected to a hub of an injector. The limiter of the injection device described in this Patent Document is formed in a tubular shape covering the periphery of a needle pipe, and has the skin contact surface from which an injection needle protrudes. The limiter determines the length of the injection needle protruding from the skin contact surface (protrusion length) to a value of 0.5 to 3.0 mm, whereby a liquid medicine injected from the injection needle is administered into the skin.

### Prior Art Documents

### [Patent Documents]

Patent Document 1: Japanese Patent Laid-open No. 2004-321826
Patent Document 2: Japanese Patent Laid-open No. 2001-137343

### [Non-patent Document]

Non-patent Document 1: R.T. Kenney et al., New England Journal of Medicine, 351, p.2295-2301 (2004)

### SUMMARY OF INVENTION

### Problem to be Solved by the Invention

In addition, in order to prevent the liquid medicine injected into the dermis from flowing back to leak out of the dermis, it has been indispensable to continue pressing the pressing section within the syringe after the pressing section is completely pressed in. However, in the injection device for administering a liquid medicine into the dermis such as the injection device described in Patent Document 2, a needle pipe with a diametral size as extremely small as 26 to 33 gauge (outside diameter: 0.2 to 0.45 mm) is commonly used. Therefore, a high pressure is generated when the liquid medicine passes through the needle pipe, and this pressure has been the cause of an increase in the injection resistance at the time of injecting the liquid medicine.

Furthermore, the dermis is a part into which a liquid medicine is more difficult to inject, as compared with ordinary blood vessels and the hypodermis; thus, an extremely great force (a force of about 20 N) has been needed to inject a liquid medicine into the dermis. As a result, a great force is required to continue pressing the pressing section, and it is difficult in the related art to inject a liquid medicine into the dermis while preventing the liquid medicine from leaking.

Taking the above-mentioned problems into account, it is an object of the present invention to provide a prefilled syringe wherein a pressing section can be held in a position reached when it has been completely pressed in so that the liquid medicine being administered into an upper layer part of skin would not leak out of the living body.

### Means for Solving Problem

In order to solve the above-mentioned problems and to attain the above object, according to the present invention, there is provided a prefilled syringe for administering a liquid medicine into a living body, including: a double-ended needle having a needlepoint capable of puncturing the living body; a needle hub for holding the double-ended needle; and an outer tube which is provided with the needle hub at an end portion thereof and has a tube hole. In addition, the prefilled syringe includes: a liquid medicine storage section which is disposed in the tube hole of the outer tube and in which the liquid medicine is stored; a sealing body for sealing the liquid medicine storage section; and a pressing section which operates the liquid medicine storage section so that the liquid medicine section can move in an axial direction of the tube hole of the outer tube. Furthermore, the prefilled syringe includes a holding section which, in a pressed state wherein the liquid medicine storage section and the pressing section have been moved toward the needle hub side until the liquid medicine stored in the liquid medicine storage section has been discharged, holds the pressed state of the pressing section.

### Effect of the Invention

According to the prefilled syringe of the present invention, the holding section for holding the pressing section is provided, whereby it is ensured that even if the force with which the pressing section is pressed is weakened, the pressing section can be held in the position reached when it has been completely pressed in. As a result, it is ensured that even where a needle pipe extremely small in diametral size for administration into the dermis is used, the liquid medicine administered can be prevented from flowing back, and the liquid medicine can be prevented from leaking out of the living body.

In addition, in the case of self-administration or the like, the pressed state has to be maintained for a while after administration of a liquid medicine. According to the prefilled syringe of the present invention, the pressing section can be held in the pressed state wherein it has been completely pressed in, and the pressed state of the pressing section can be maintained simply.

### BRIEF DESCRIPTION OF DRAWINGS

FIG. 1 is a sectional view showing a first embodiment of the prefilled syringe according to the present invention.
FIG. 2 is a sectional view showing a pressed state in the first embodiment of the prefilled syringe according to the present invention.
FIG. 3 is a sectional view showing a second embodiment of the prefilled syringe according to the present invention.
FIG. 4 is a sectional view showing a pressed state in the second embodiment of the prefilled syringe according to the present invention.
FIG. 5 is a sectional view showing a third embodiment of the prefilled syringe according to the present invention.
FIG. 6 is a sectional view showing a pressed state in the third embodiment of the prefilled syringe according to the present invention.
FIG. 7 is a sectional view showing an essential part of a fourth embodiment of the prefilled syringe according to the present invention.
FIG. 8 is a sectional view showing an essential part of a pressed state in the fourth embodiment of the prefilled syringe according to the present invention.

### MODE FOR CARRYING OUT THE INVENTION

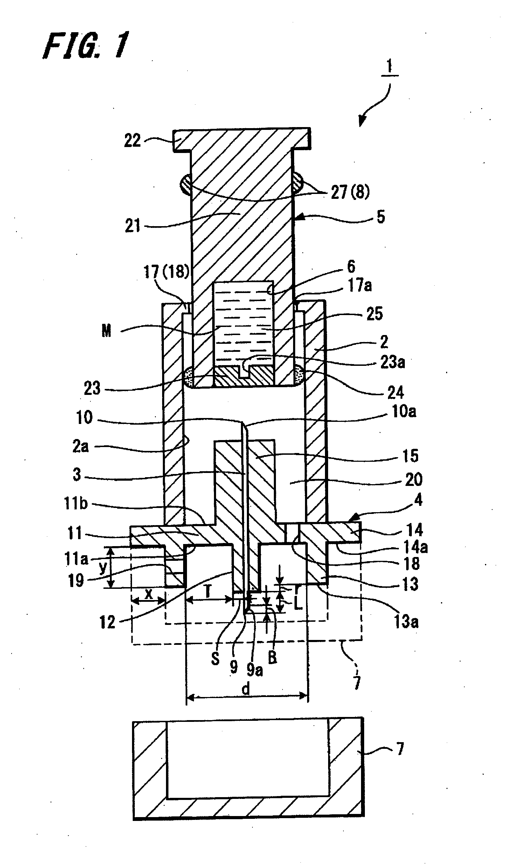

Hereunder, embodiments of the prefilled syringe according to the present invention will be described below, referring to FIGS. 1 to 8. Incidentally, in the drawings, common members are denoted by the same reference letters or numerals. Besides, the present invention is not restricted to the following embodiments.

Incidentally, description will be made in the following order.

### 1. First Embodiment

1-1. Configuration Example of Prefilled Syringe
1-2. Method of using Prefilled Syringe
2. Second Embodiment
3. Third Embodiment
4. Fourth Embodiment

### <1. First Embodiment>

### 1-1. Configuration Example of Prefilled Syringe

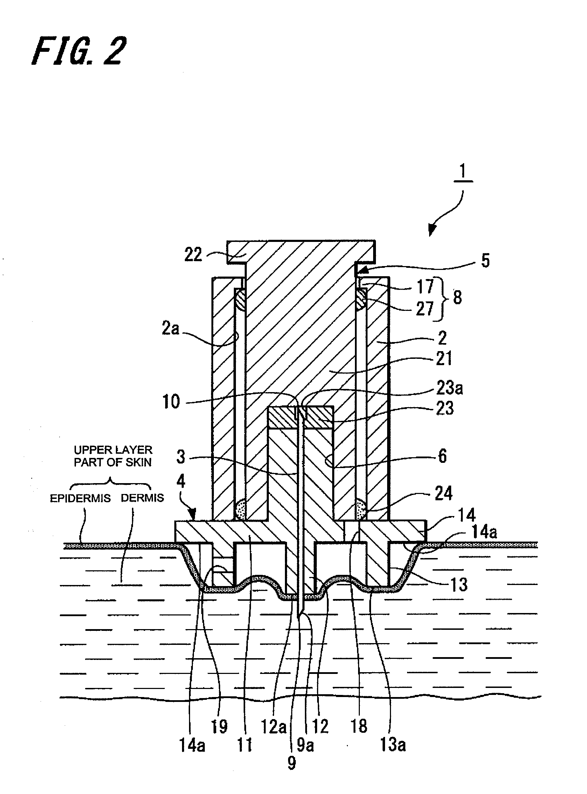

First, referring to FIGS. 1 and 2, a prefilled syringe according to a first embodiment (hereinafter referred to as "this embodiment") of the present invention will be described.

FIG. 1 is a sectional view showing a prefilled syringe in this embodiment, and FIG. 2 is a sectional view showing a pressed state in this embodiment.

The prefilled syringe 1 in this embodiment contains a liquid medicine M preliminarily stored therein to be administered into a living body, and is a syringe for administering the liquid medicine M into an upper layer part of skin of the living body. As shown in FIG. 1, the prefilled syringe 1 includes an outer tube 2, a hollow needle pipe 3 having a needle hole, a needle hub 4 for holding the needle pipe 3, a liquid medicine container 5, a cap 7, and a holding section 8.

Examples of the liquid medicine M preliminarily stored in the prefilled syringe 1, in this embodiment, include various vaccines for prevention of various infectious diseases such as influenza, but are not limited to the vaccines. Other examples of the liquid medicine M than vaccines include carbohydrate injections involving glucose, etc., electrolyte replenisher injections for correction involving sodium chloride, or potassium lactate, etc., vitamin preparations, antibiotic injections, radiopaque materials, steroid preparations, protease inhibitors, fat emulsions, carcinostatic agents, anesthetic agents, stimulants, narcotic drugs, heparin calcium, and immuno antibody medicines.

### [Outer Tube]

### First, the outer tube 2 will be described.

The outer tube 2 is formed in a hollow cylindrical shape provided with a tube hole 2a substantially in the center thereof. The outer tube 2 is opening at both axial-directional ends thereof. In addition, the needle hub 4 is so disposed as to close the opening at one axial-directional end of the outer tube 2.

In addition, a stopper 17 is provided at the other axial-directional end of the outer tube 2. The stopper 17 is an inner flange which projects radially inward from the tube hole 2a of the outer tube 2. Further, the stopper 17 is formed with a through-hole 17a to be penetrated by the liquid medicine container 5 which will be described later.

Examples of the material for the outer tube 2 include various resins such as polyvinyl chloride, polyethylene, polypropylene, cyclic polyolefins, polystyrene, poly(4-methylpentene-1), polycarbonates, acrylic resins, acrylonitrile-butadiene-styrene copolymer, polyesters such as polyethylene terephthalate, etc., butadiene-styrene copolymer, and polyamides (e.g., nylon 6, nylon 6,6, nylon 6,10, or nylon 12). Among these materials, such reins as polypropylene, cyclic polyolefins, polyesters and poly(4-methylpentene-1) are preferred because of their easily moldable properties. Incidentally, it is preferable for the material of the outer tube 2 to be substantially transparent, for securing inside visibility.

### [Needle Pipe]

### Next, the needle pipe 3 will be described below.

As the needle pipe 3, those of a size of 22 to 33 gauge (outside diameter: 0.2 to 0.7 mm) according to ISO medical needle pipe standard (ISO9625: 1991/Amd. 1:2001 (E)) can be used. Incidentally, in the case for use in administering into an upper layer part of skin, there can be used needle pipes of 26 to 33 gauge, preferably 30 to 33 gauge.

The needle pipe 3 is provided at one end thereof with a first needlepoint 9 for puncturing a living body, and is provided at the other end thereof with a second needlepoint 10 for puncturing a sealing body of the liquid medicine storage section 6. Thus, the needle pipe 3 is a double-ended needle. The first needlepoint 9 has a cutting edge surface 9a. It suffices that the length of the cutting edge surface 9a measured along the extending direction of the needle pipe 3 (this length will hereafter be referred to as "bevel length B") is not more than the smallest thickness of the upper layer part of skin (which will be described later) of 1.4 mm (adult). In addition, it suffices for the bevel length B to be not less than about 0.5 mm, which is the bevel length in the case where a 33 gauge needle pipe is formed with a short bevel. Thus, the bevel length B is preferably set in the range of 0.5 to 1.4 mm.

Furthermore, it is more preferable that the bevel length B is not more than the smallest thickness of the upper layer part of skin of 0.9 mm (child). Thus, the bevel length B is more preferably set in the range of 0.5 to 0.9 mm. Incidentally, the "short bevel" means a cutting edge surface at an angle of 18 to 25 degrees against the longitudinal direction of the needle, which is commonly used for injection needles.

The second needlepoint 10 has a cutting edge surface 10a. The length of the cutting edge surface 10a measured along the extending direction of the needle pipe 3 may be set arbitrarily, and may be set to be equal to the length of the cutting edge surface 9a of the first needlepoint 9. In addition, the needle pipe 3 has its intermediate portion held by the needle hub 4.

Examples of the material which can be used to form the needle pipe 3 include stainless steel, aluminum, aluminum alloys, titanium, titanium alloys and other metals. Besides, as the needle pipe 3, there can be applied straight needles and taper needles which have a taper structure at least at part thereof. It suffices for the taper needle to have a configuration wherein its outside diameter on the side of the second needlepoint 10 is greater than its outside diameter on the side of the first needlepoint 9, and to have a taper structure at its intermediate portion. Incidentally, in this case, the first needlepoint 9 and the second needlepoint 10 are different in shape.

### [Needle Hub]

Next, the needle hub 4 which holds the needle pipe 3 will be described below.

The needle hub 4 includes a roughly circular disk-shaped hub main body 11, an adjustment section 12, a stabilization section 13, a guide section 14 as a pressing yardstick section, and a sealing body contact section 15. The needle hub 4 is so disposed as to close an opening on one end side of the outer tube 2 in the state of holding the needle pipe 3. In this instance, the second needlepoint 10 of the needle pipe penetrating the needle hub 4 is disposed in an internal space 20 defined by the tube hole 2a of the outer tube 2 and the needle hub 4.

The hub main body 11 is provided on one end side thereof with the adjustment section 12 and the stabilization section 13, and is provided on the other end side thereof with the sealing body contact section 15. The material for the needle hub 4 is not particularly restricted; for example, the same materials as those for the outer tube 2 described above can be used. Furthermore, the needle hub 4 may be formed integrally with the outer tube 2.

In addition, the hub main body 11 is provided therein with a first vent hole 18 penetrating it from one end side to the other end side thereof. Through the first vent hole 18, the internal space 20 surrounded by the tube hole 2a of the outer tube 2 and the needle hub 4 is let communicate with the outside of the internal space 20. Further, a filter may be provided in the first vent hole 18, in order to enhance retention in a sterile state of the internal space 20 surrounded by the tube hole 2a of the outer tube 2 and the needle hub 4.

Incidentally, while this embodiment wherein the number of the first vent hole(s) 18 is one has been described in this embodiment, this is not restrictive; thus, the hub main body 11 may be formed with a plurality of first vent holes 18.

Next, the adjustment section 12 will be described below.

The adjustment section 12 is provided at a central portion of one end face 11 a on one side of the hub main body 11, and is configured as a projection projecting in the axial direction of the hub main body 11. The axis of the adjustment section 12 coincides with the axis of the hub main body 11. In addition, the adjustment section 12 is penetrated by the needle pipe 3. Besides, an end face of the adjustment section 12 is a needle protrusion surface 12a from which the first needlepoint 9 side of the needle pipe 3 protrudes.

The needle protrusion surface 12a is formed as a flat surface orthogonal to the axial direction of the needle pipe 3. When the needle pipe 3 is let puncture an upper layer part of skin, the needle protrusion surface 12a makes contact with the surface of the skin, to determine the depth of puncture of the needle pipe 3. In other words, the depth to which the needle pipe 3 punctures the upper layer part of skin is determined by the length by which the needle pipe 3 protrudes from the needle protrusion surface 12a (this length will hereafter be referred to as "protrusion length L").

The thickness of the upper layer part of skin corresponds to the depth from the surface of skin to a dermal layer (dermis), and is generally in the range of 0.5 to 3.0 mm. Accordingly, the protrusion length L of the needle pipe 3 can be set in the range of 0.5 to 3.0 mm.

Meanwhile, vaccines are generally administered into a brachial region, and, in consideration of administration into an upper layer part of skin, it is considered suitable for the vaccine to be administered into a peripheral region of shoulder where the skin is thick, particularly into a deltoid region.

In view of this, 19 children and 31 adults were subjected to measurement of the thickness of respective skin upper layer part of the deltoid muscle. The measurement was conducted by imaging the skin upper layer part where an ultrasonic wave reflectance is high, by use of an ultrasonic measuring instrument (NP60R-UBM, a high-resolution echoscope for small animals, produced by Nepa Gene Co., Ltd.). Incidentally, since the measurements were in log-normal distribution, the range of MEAN±2SD was determined by use of geometric mean.

As a result, the thickness of the skin upper layer part of the deltoid muscle in children was found to be 0.9 to 1.6 mm. Besides, the thickness of the skin upper layer part of the deltoid muscle in adults was found to be 1.4 to 2.6 mm in distal part, 1.4 to 2.5 mm in central part, and 1.5 to 2.5 mm in proximal part. From these results, it was confirmed that the thickness of the skin upper layer part of the deltoid muscle is not less than 0.9 mm in the cases of children, and not less than 1.4 mm in the cases of adults. Accordingly, in injection into a skin upper layer part of the deltoid muscle, the protrusion length L of the needle pipe 3 is preferably set in the range of 0.9 to 1.4mm.

With the protrusion length L set in this manner, the cutting edge surface 9a of the first needlepoint 9 can be located assuredly in the upper layer part of skin. As a result, the needle hole (liquid medicine outlet) opening at the cutting edge surface 9a can be arrived in the upper layer part of skin, irrespectively of at which position of the needle hole in the cutting edge surface 9a is located. Incidentally, even when the liquid medicine outlet is located in the upper layer part of skin, if the first needlepoint 9 pierces too deeply into the upper layer part of skin, the liquid medicine M would be seeped under a subcutaneous portion through the part between the side surface of an end portion of the first needlepoint 9 and the incised skin. Therefore, it is important for the cutting edge surface 9a to be located assuredly in the upper layer part of skin.

Incidentally, in the case of use for administration into an upper layer part of skin, it is difficult to set the bevel length B to or below 1.0 mm, for needle pipes of more than 26 gauge in diametral size. Therefore, in order to set the protrusion length L of the first needlepoint 9 of the needle pipe 3 in the preferable range (0.9 to 1.4 mm), it is preferable to use a needle pipe smaller than 26 gauge in diametral size.

The needle protrusion surface 12a is formed so that the distance S from the circumferential edge thereof to the circumferential surface of the needle pipe 3 is not more than 1.4 mm, and is preferably in the range of 0.3 to 1.4 mm. The distance S from the circumferential edge of the needle protrusion surface 12a to the circumferential surface of the needle pipe 3 is set taking into account the fact that a pressure is exerted on a blister formed by administration of the liquid medicine into the upper layer part of skin. Specifically, the needle protrusion surface 12a is set to a size which is sufficiently smaller than the blister formed in the upper layer part of skin and which, therefore, does not obstruct the formation of the blister. Consequently, it is possible to prevent a situation in which the needle protrusion surface 12a would press the skin in the surroundings of the needle pipe 3 to cause leakage of the liquid medicine administered.

Next, the stabilization section 13 will be described below.

The stabilization section 13 is provided at one end face 11 a of the hub main body 11. The stabilization section 13 is formed in the shape of a tube continuous with a circumferential edge portion of the one end face 11a. In a tube hole of the stabilization section 13 are disposed the first needlepoint 9 of the needle pipe 3 and the adjustment section 12. In other words, the stabilization section 13 is formed in the shape of a tube which covers the periphery of the adjustment section 12 penetrated by the needle pipe 3.

In addition, when the first needlepoint 9 of the needle pipe 3 is let puncture a living body, as shown in FIG. 2, the needle protrusion surface 12a makes contact with the surface of the skin, and the latter makes contact with an end face 13a of the stabilization section 13. In this instance, the contact of the end face 13a of the stabilization section 13 with the skin stabilizes the prefilled syringe 1, whereby the needle pipe 3 can be maintained in the posture of being substantially perpendicular to the skin.

Incidentally, the needle pipe 3 can be held in the posture of being substantially perpendicular to the skin, even when the end face 13a of the stabilization section 13 is located on the same plane as the needle protrusion surface 12a or is located on the side of the first needlepoint 9 of the needle pipe 3 as compared with the needle protrusion surface 12a. Incidentally, taking into account the protuberance of the skin upon pressing of the stabilization section 13 against the skin, the axial distance r between the end face 13a of the stabilization section 13 and the needle protrusion surface 12a is preferably set to be not more than 1.3 mm.

In addition, the inside diameter d of the stabilization section 13 is set to be equal to or greater than the diameter of the blister formed in the skin. Specifically, the inside diameter d is so set that the distance T from the inner wall surface of the stabilization section 13 to the circumferential edge of the needle protrusion surface 12a is in the range of 4 to 15 mm. This makes it possible to prevent the formation of the blister from being obstructed by application of a pressure to the blister from the inner wall surface of the stabilization section 13.

The distance T from the inner wall surface of the stabilization section 13 to the circumferential edge of the needle protrusion surface 12a does not have any particular upper limit, insofar as it is not less than 4 mm. If the distance T is enlarged, however, the outside diameter of the stabilization section 13 is enlarged, so that in the case of puncturing a slender arm such as a child's arm with the needle pipe 3, it is difficult to bring the whole part of the end face 13a of the stabilization section 13 into contact with the skin. Therefore, the distance T is preferably determined to be 15 mm at maximum, taking into account the slenderness of the child's arm.

In addition, where the distance S from the circumferential edge of the needle protrusion surface 12a to the circumferential surface of the needle pipe 3 is not less than 0.3 mm, the adjustment section 12 would not enter into the skin. Therefore, taking into account the distance T (not less than 4 mm) from the inner wall surface of the stabilization section 13 to the circumferential edge of the needle protrusion surface 12a and the diameter (about 0.3 mm) of the needle protrusion surface 12a, the inside diameter d of the stabilization section 13 can be set to be not less than 9 mm.

Furthermore, the stabilization section 13 is formed with a second vent hole 19 penetrating the stabilization section 13 from the outer circumferential surface to the inner circumferential surface of the latter. With the second vent hole 19 thus provided in the stabilization section 13, it is ensured that when the stabilization section 13 is set in contact with a skin, as shown in FIG. 2, the space surrounded by the stabilization section 13 and the skin and the space outside the stabilization section 13 can be let communicate with each other. Besides, the first vent hole 18 and the second vent hole 19 constitute a ventilation means for opening the internal space 20 to the exterior.

Incidentally, the shape of the stabilization section 13 is not restricted to the hollow cylindrical shape; for example, the stabilization section 13 may be formed in a polygonal tubular shape, such as a tetragonal prism or hexagonal prism provided with a tube hole in the center thereof.

Next, the guide section 14 as a pressing yardstick section will be described below.

The guide section 14 is provided at a side surface portion of the hub main body 11. The guide section 14 is formed as a flange having a ring-like shape projecting outward in the radial direction of the hub main body 11 from a side surface portion of the hub main body 11. In addition, the guide section 14 is projecting substantially perpendicularly to the outer circumferential surface of the stabilization section 13.

Furthermore, the guide section 14 has a contact surface 14a coming into contact with a skin. The contact surface 14a is a flat surface which is substantially parallel to the end face 13a of the stabilization section 13. With the stabilization section 13 pressed against a skin until the contact surface 14a of the guide section 14 makes contact with the skin, the force with which the stabilization section 13 and the needle pipe 3 presses the skin can always be secured to be not less than a predetermined value. This ensures that the part (corresponding to the protrusion length L), protruding from the needle protrusion surface 12a, of the needle pipe 3 is made to puncture the skin assuredly.

In addition, the distance from the contact surface 14a of the guide section 14 to the end face 13a of the stabilization section 13 is set to such a length that the needle pipe 3 can puncture a skin, with the stabilization section 13 and the needle pipe 3 pressed against the skin with an appropriate pressing force. Hereafter, this length will be referred to as "guide section height y."

Incidentally, the appropriate pressing force with which the needle pipe 3 and the stabilization section 13 are pressed against the skin is, for example, 3 to 20 N. As a result, the pressing force exerted on the skin by the needle pipe 3 and the stabilization section 13 can be guided for the user by the guide section 14, and the first needlepoint 9 and the cutting edge surface 9a of the needle pipe 3 can be assuredly located in the upper layer part of skin, which is effective in making the user feel safe.

Specifically, in the case where the inside diameter d of the stabilization section 13 is in the range of 11 to 14 mm, the guide section height y is appropriately set based on the length x (hereafter referred to as "guide section length x") from the projecting end face of the guide section 14 to the outer circumferential surface of the stability section 13. For instance, in the case where the inside diameter d of the stabilization section 13 is 12 mm, the guide section height y is set in the range of 2.3 to 6.6 mm when the guide section length x is 3.0 mm, for example.

Next, the sealing body contact section 15 will be described below.

The sealing body contact section 15 is provided at a central portion of the other end face 11b, opposite to the one end face 11a, of the hub main body 11. The sealing body contact section 15 is configured as a projection projecting in a substantially cylindrical shape along the axial direction of the hub main body 11. In addition, the sealing body contact section 15 is penetrated by the needle pipe 3, and the second needlepoint 10 of the needle pipe 3 is protruding from an end face of the sealing body contact section 15.

In addition, in this embodiment, the axis of the needle pipe 3 and the axes of the adjustment section 12 and the sealing body contact section 15 coincide with each other. However, even when the axis of the needle pipe 3 and the axis of the sealing body contact section 15 do not coincide with each other, their purpose can be fulfilled. Furthermore, while an example wherein the sealing body contact section 15 is formed in a substantially cylindrical shape has been described, the shape of the sealing body contact section 15 is not restricted to the substantially cylindrical shape. It suffices for the sealing body contact section 15 to be in such a shape as to press a sealing body 23 which will be described later; for example, the sealing body contact section 15 may be formed in a substantially semi-circular or prismatic shape.

The sealing body contact section 15 is disposed inside the internal space 20 defined by the tube hole 2a of the outer tube 2 and the needle hub 4 when the opening on one end side of the outer tube 2 is closed with the needle hub 4.

### [Liquid Medicine Container]

Next, the liquid medicine container 5 will be described below.

The liquid medicine container 5 has a substantially cylindrical container main body 21, and a pressing section 22 to be pressed by the user. The liquid medicine container 5 penetrates the through-hole 17a of the stopper 17, and is inserted in the tube hole 2a of the outer tube 2. In addition, one end side of the container main body 21 is formed as the pressing section 22, and the other end side of the container main body 21 is formed as the liquid medicine storage section 6. Besides, the diameter of the container main body 21 is set smaller than the diameter of the tube hole 2a of the outer tube 2.

The pressing section 22 is provided on the axial-directionally one end side of the container main body 21. In addition, the pressing section 22 is formed as a flange spreading outward in the radial direction of the container main body 21. With the pressing section 22 pressed by the user, the container main body 21 is moved within the tube hole 2a of the outer tube 2 along the axial direction of the latter. In other words, in this embodiment, the liquid medicine container 5 functions also as a pusher by which the liquid medicine stored in the liquid medicine storage section 6 is pushed out.

Besides, the container main body 21 is provided with a lock section 27 at an outer circumferential surface thereof. The lock section 27 is disposed in the vicinity of the pressing section 22 of the container main body 21. The lock section 27 is formed continuously along the circumferential direction of the outer circumferential surface of the container main body 21. The lock section 27 is a projection projecting in a substantially semi-circular shape from the outer circumferential surface of the container main body 21.

As shown in FIG. 2, when the liquid medicine container 5 is pushed in along the axial direction of the tube hole 2a of the outer tube 2 to the needle hub 4 side, the lock section 27 is locked by the stopper 17 of the outer tube 2. The lock section 27 and the stopper 17 constitute the holding section 8 for holding the liquid medicine container 5.

Furthermore, a holding force generated by locking between the lock section 27 and the stopper 17 is set to be greater than the injection resistance at the time of injecting the liquid medicine, and is set to be not less than 3 N, for example. Incidentally, the holding force is preferably set to be not less than 5 N, in order to hold the pressure with which the pressing section 22 is pressed at the time of self-injection or the like. Besides, the holding force is more preferably set to be not less than 20 N, in order to resist against the injection resistance. This ensures that when the pressing section 22 is completely pressed in, the pressure-holding state can be maintained and the pressing section 22 can be prevented from being pushed back by the injection resistance.

In addition, the stopper 17 may be formed from a flexible material. As a result, it is ensured that when the liquid medicine container 5 is pushed into the tube hole 2a of the outer tube 2, the lock section 27 can ride over the stopper 17 smoothly. Incidentally, in the case where the holding force generated by the lock section 27 and the stopper 17 is to be further enhanced, the stopper 17 is preferably formed from a hard material. Furthermore, the stopper 17 may be provided continuously in the axial direction of the tube hole 2a of the outer tube 2, or a plurality of the lock sections 27 may be provided in the axial direction of the container main body 21.

Incidentally, while an example wherein the lock section 27 is formed continuously along the circumferential direction of the outer circumferential surface of the container main body 21 has been described in this embodiment, this is not restrictive. For instance, the lock section 27 may be formed intermittently at the outer circumferential surface of the container main body 21. Furthermore, the sectional shape of the lock section 27 is not limited to the roughly semi-circular shape; for example, the sectional shape may be a roughly triangular shape or a roughly tetragonal shape.

### [Liquid Medicine Storage Section]

Next, the liquid medicine storage section 6 will be described below.

The liquid medicine storage section 6 is provided on the axial-directionally other end side of the container main body 21. The liquid medicine storage section 6 is a recess recessed in a substantially cylindrical shape from the axial-directionally other end face of the container main body 21. The liquid medicine M is stored in the liquid medicine storage section 6. In addition, the diameter of the aperture of the liquid medicine storage section 6 is set to be approximately equal to or slightly greater than the diameter of the sealing body contact section 15.

Furthermore, the liquid medicine storage section 6 is fitted with the sealing body 23 so as to close the aperture thereof. Therefore, the liquid medicine M is sealed within a liquid medicine space 25 surrounded by the liquid medicine storage section 6 and the sealing body 23. The liquid medicine space 25 is a hermetically closed space which is sealed in an air-tight manner by the sealing body 23, and is kept in a sterile state. The liquid medicine M is stored in the tube hole 2a of the outer tube 2 in the state of being sealed in the liquid medicine storage section 6.

In addition, as shown in FIG. 2, the sealing body 23 is slid within the liquid medicine storage section 6 in the axial direction by being pressed by the sealing body contact section 15 of the needle hub 4 at the time of use of the prefilled syringe 1. Then, the sealing body 23 is punctured by the second needlepoint 10 of the needle pipe 3.

Besides, that face of the sealing body 23 which faces a deep end face of the liquid medicine storage section 6 is formed in its substantially central area with a needle accommodating recess 23a continuous with a hole pierced by the second needlepoint 10. That section of the needle accommodating recess 23a which is orthogonal to the axial direction of the sealing body 23 is formed to be greater than the outside diameter of the needle pipe 3. In the condition where administration of the liquid medicine is completed, the second needlepoint 10 side of the needle pipe 3 is disposed within the needle accommodating recess 23a.

Incidentally, the amount of the liquid medicine M stored in the liquid medicine space 25 (the volume of the liquid medicine space 25) is not particularly limited; for example, it is preferably about 0.02 to 2.0 mL, more preferably about 0.05 to 0.8 mL. In other words, the prefilled syringe 1 is particularly suitable for the case of administering such a small amount of liquid medicine.

In addition, the material of the sealing body 23 is not particularly restricted, but it is preferable for the sealing body 23 to be formed from an elastic material so as to enhance liquid-tightness of the liquid medicine storage section 6. Examples of the material which can be used to form the sealing body 23 include elastic materials such as various rubber materials exemplified by natural rubber, butyl rubber, isoprene rubber, butadiene rubber, styrene-butadiene rubber, silicone rubbers, isoprene rubber, etc., various thermoplastic elastomers exemplified by polyurethanes, polyesters, polyamides, polyolefins, or polystyrenes, and their mixtures.

Incidentally, it suffices for the sealing body 23 to have at least its outer circumferential portion formed from the elastic material as above-mentioned. For instance, the sealing body 23 may have a configuration wherein a core portion (not shown) is formed of a resin material and the above-mentioned elastic material is disposed so as to cover the outer circumference of the core portion.

The material of the liquid medicine container 5 configured as above-mentioned is not specifically restricted; for instance, the same materials as those for the outer tube 2 mentioned above can be used to form the liquid medicine container 5. Incidentally, the material of the liquid medicine container 5 is preferably substantially transparent, in order that inside visibility can be secured. Besides, graduations may be formed on the outer circumferential surface of the liquid medicine storage section 6 of the liquid medicine container 5. This permits the user to grasp the amount of the liquid medicine M stored inside the liquid medicine storage section 6.

Furthermore, a seal member 24 is attached to the outer circumferential surface, on the liquid medicine storage section 6 side, of the container main body 21. The seal member 24 is an O-ring, which is provided continuously along the circumferential direction of the outer circumferential surface of the container main body 21. In addition, the seal member 24 and the container main body 21 may be formed by two-color molding.

The seal member 24 is put in close contact with the outer circumferential surface of the container main body 21 and the inner wall of the tube hole 2a when the liquid medicine container 5 is inserted in the tube hole 2a of the outer tube 2. Therefore, perfect sealing is attained between the tube hole 2a of the outer tube 2 and the container main body 21. As a result, in an unused state, the internal space 20 surrounded by the tube hole 2a of the outer tube 2 and the hub main body 11 of the needle hub 4 is sealed in an air-tight manner by the seal member 24. In addition, the close contact of the seal member 24 with the outer circumferential surface of the container main body 21 and the inner wall of the tube hole 2a enables the container main body 21 to be stably held at an intermediate portion of the tube hole 2a of the outer tube 2.

The material of the seal member 24 is not particularly limited, and the same materials as those for the sealing body 23 can be used to form the seal member 24.

### [Cap]

Next, the cap 7 will be described below.

The cap 7 is a member for sealing a space that surrounds the first needlepoint 9 of the needle pipe 3 as well as the adjustment section 12 and the stabilization section 13 of the needle hub 4. In the unused state, the cap 7 is detachably mounted to the stabilization section 13 of the needle hub 4.

The cap 7 is formed in the shape of a bottomed tube which has a bottom portion on the distal side; in this embodiment, the cap 7 is formed in a bottomed hollow cylindrical shape. In the unused state, an inside surface of the cap 7 is mounted to the outer circumferential surface of the stabilization section 13, whereby the second vent hole 19 and the inside of the cap 7 are hermetically closed. Consequently, the internal space 20 and the inside of the cap 7 are maintained in a sterile state.

At the time of using the prefilled syringe 1, the cap 7 is detached from the needle hub 4, whereby sealing of the first needlepoint 9 of the needle pipe 3 is released. In addition, the second vent hole 19 in the needle hub 4 is let open. Consequently, in the condition where the stabilization section 13 is in contact with a skin, as shown in FIG. 2, the space surrounded by the stabilization section 13 and the skin and the internal space 20 can be opened to the exterior through the first vent hole 18 and the second vent hole 19.

Besides, the material of the cap 7 is not specifically restricted; for example, the same materials as those for the outer tube 2 mentioned above or those for the liquid medicine container 5 mentioned above may be used to form the cap 7.

### 1-2. Method of Using Prefilled Syringe

Next, the method of using the prefilled syringe 1 according to this embodiment will be described below, referring to FIGS. 1 and 2.

First, as shown in FIG. 1, the cap 7 is detached from the stabilization section 13 of the needle hub 4. As a result, sealing of the first needlepoint 9 of the needle pipe 3 is released, and the second vent hole 19 in the needle hub 4 is let open. Then, the end face 13a of the stabilization section 13 is made to face a skin. The first needlepoint 9 of the needle pipe 3 is made to face the skin being to be punctured by injection whereby. Next, as shown in FIG. 2, the prefilled syringe 1 is moved substantially perpendicularly to the skin, to let the needle pipe 3 puncture the skin and to press the end face 13a of the stabilization section 13 against the skin.

In this instance, the needle protrusion surface 12a of the adjustment section 12 and the end face 13a of the stabilization section 13 are located on the same plane. This ensures that the needle protrusion surface 12a of the adjustment section 12 makes contact with the skin, whereby the skin can be deformed to be flat, and the first needlepoint 9 of the needle pipe 3 can puncture the skin by the protrusion length L.

Subsequently, the stabilization section 13 is pressed against the skin until the contact surface 14a of the guide section 14 makes contact with the skin. Here, the guide section height y is set to such a length that the puncturing of the skin can be achieved with the needle pipe 3 and the stabilization section 13 pressed against the skin with an appropriate pressing force. This ensures that the force with which the stabilization section 13 is pressed against the skin is brought to a predetermined value. Therefore, the pressing force of the stabilization section 13 can be guided for the user, and the stabilization section 13 can be pressed against the skin with an appropriate pressing force. Consequently, the first needlepoint 9 of the needle pipe 3 and the cutting edge surface 9a can be assuredly located within the upper layer part of skin.

With the guide section 14 thus serving as a mark in guiding the pressing force of the stabilization section 13, the first needlepoint 9 of the needle pipe 3 can be reliably located in the upper layer part of skin. Therefore, the liquid medicine can be administered into the upper layer part of skin, and the user's feeling of security can be enhanced.

In addition, the contact of the stabilization section 13 with the skin makes it possible to stabilize the needle pipe 3, and to let the needle pipe 3 puncture the skin straight. Consequently, unintentional movement of the needle pipe 3 can be prevented from occurring, and stable administration of the liquid medicine can be performed.

Furthermore, in the case of a needle having an extremely small protrusion length of about 0.5 mm, for example, the first needlepoint 9 put into contact with a skin may fail to puncture the skin. However, when the stabilization section 13 is pressed against the skin and the skin is pressed down in the vertical direction, the skin on the inner side of the stabilization section 13 is stretched, resulting in the condition where a tension is applied to the skin. Therefore, it becomes difficult for the skin to escape from the first needlepoint 9 of the needle pipe 3, and, accordingly, it becomes easier for the first needlepoint 9 to pierce the skin.

Besides, since the protrusion length L is set in the range of 0.5 to 3.0 mm, the first needlepoint 9 of the needle pipe 3 and the cutting edge surface 9a are assuredly located in the upper layer part of skin. The adjustment section 12 is fixed in close contact with the surroundings of the needle pipe 3, and it is ensured that no gap is generated between the adjustment section 12 and that portion of the needle pipe 3 which penetrates the adjustment section 12.

Therefore, when the needle protrusion surface 12a of the adjustment section 12 is put in contact with the skin, the skin in the surroundings of the needle pipe 3 can be deformed to be flat. As a result, the needle pipe 3 can be made to puncture the skin by the protrusion length L, and the first needlepoint 9 of the needle pipe 3 can be assuredly located in the upper layer part of skin.

Next, the user presses the pressing section 22 of the liquid medicine container 5, whereby the container main body 21 and the liquid medicine storage section 6 with the liquid medicine M confined therein are slid within the tube hole 2a along the axial direction of the outer tube 2. Then, the sealing body contact section 15 of the needle hub 4 makes contact with the sealing body 23. In this instance, the second needlepoint 10 of the needle pipe 3 is let puncture the sealing body 23. As a result, passage of the liquid medicine M stored in the liquid medicine storage section 6 into and through the needle pipe 3 is completed.

In addition, the needle hub 4 is provided with the first vent hole 18 and the second vent hole 19. At the time of an operation of moving the container main body 21, air in the internal space 20 is discharged to the exterior via the first vent hole 18 and the second vent hole 19. Therefore, the pressure in the internal space 20 and the space surrounded by the stabilization section 13 and the skin can be prevented from being raised. Accordingly, the operation of moving the liquid medicine container 5 can be performed readily and smoothly.

Subsequently, the pressing section 22 is pressed toward the needle hub 4 side of the outer tube 2, whereon the sealing body 23 is pushed into the liquid medicine storage section 6 by the sealing body contact section 15. Then, the sealing body 23 is slid within the liquid medicine storage section 6 along the axial direction of the latter, whereby the liquid medicine M in the liquid medicine storage section 6 is discharged through the second needlepoint 10 of the needle pipe 3 and then through the first needlepoint 9 thereof into the living body.

Furthermore, the lock section 27 of the liquid medicine container 5 is locked by the stopper 17 provided on the outer tube, and the container main body 21 is held at a predetermined position in the outer tube 2, specifically, at a position reached when the container main body 21 is completely pressed to the needle hub 4. Here, the holding force of the holding section 8 constituted of the lock section 27 and the stopper 17 is set to be greater than the injection resistance. Therefore, even if the force with which the pressing section 22 is pressed is weakened after completion of the administration of the liquid medicine M, the liquid medicine container 5 can be prevented from moving toward the side of the other end portion of the outer tube 2. Furthermore, with the pressing section 22 completely pressed in, a predetermined amount of the liquid medicine M can be reliably administered into the living body.

Besides, the second needlepoint 10 is accommodated in the needle accommodating recess 23a, and does not protrude from the surface of the sealing body 23 to the side of the deep end face of the liquid medicine container 5. Therefore, the sealing body 23 makes contact with the deep end face of the liquid medicine container 5, without leaving any gap therebetween.

This ensures that the pressing section 22 can be restrained from being pushed back, from the position reached upon full pushing of the pressing section 22 and the sealing body 23, by the pressure (injection resistance) generated at the time of passage of the liquid medicine M through the needle pipe 3 or by the force of the liquid medicine M tending to flow back from the living body. Consequently, the liquid medicine M administered into the living body can be prevented from leaking or flowing back into the liquid medicine storage section 6.

Here, since the needle protrusion surface 12a of the adjustment section 12 and the inside diameter d of the stabilization section 13 are set to appropriate sizes, the liquid medicine injected into the living body can be prevented from leaking out of the living body, and the liquid medicine can be assuredly administered into the upper layer part of skin. Consequently, administration of the liquid medicine M by use of the prefilled syringe 1 according to this embodiment is completed.

### <2. Second Embodiment>

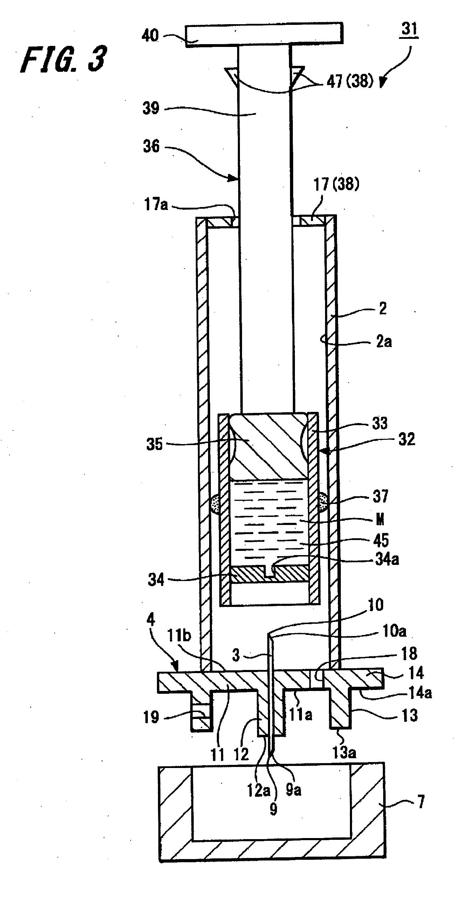

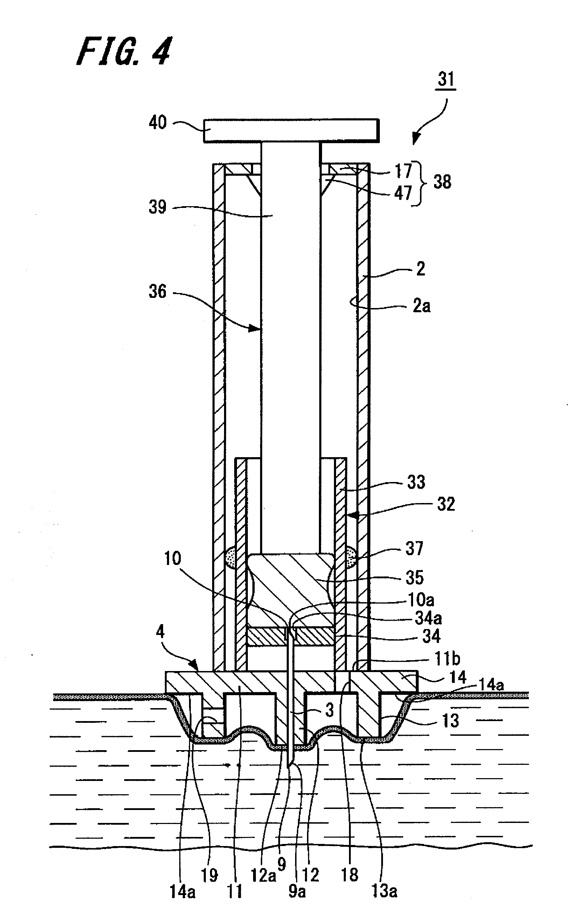

Next, a second embodiment of the prefilled syringe according to the present invention will be described below, referring to FIGS. 3 and 4.

FIGS. 3 and 4 are sectional views showing a prefilled syringe according to the second embodiment.

The prefilled syringe 31 according to the second embodiment differs from the prefilled syringe 1 according to the first embodiment in that a liquid medicine storage section and a pressing section are configured as separate members, a pusher having the pressing section is provided, and the sealing body contact section 15 is eliminated from the needle hub 4. Here, therefore, the liquid medicine container and the pressing section will be described, and the same parts as those in the above-described prefilled syringe 1 are denoted by the same reference letters or numerals as used above, and overlapping descriptions of the parts will be omitted.

As shown in FIG. 4, a liquid medicine storage section 32 includes a substantially hollow cylindrical tube body 33 and a first sealing body 34. The tube body 33 is opening at both axial-directional ends thereof. The tube body 33 is inserted in a tube hole 2a of an outer tube 2, from an aperture on the other end side of the outer tube 2. The tube body 33 is pushed out by a pusher 36 (which will be described later), thereby being slid within the tube hole 2a in the axial direction of the latter.

The first sealing body 34 is so disposed as to close an aperture on the axial-directionally one end side of the tube body 33. When the tube body 33 is inserted in the tube hole 2a of the outer tube 2, the first sealing body 34 faces a second needlepoint 10 of a needle pipe 3 held by a needle hub 4.

In addition, that surface of the first sealing body 34 which faces a second sealing body 35 (described later) is formed in its substantially central area with a needle accommodating recess 34a continuous with a hole pierced by the second needlepoint 10. That section of the needle accommodating recess 34a which is orthogonal to the axial direction of the first sealing body 34 is formed to be greater than the outside diameter of the needle pipe 3. In the condition where administration of a liquid medicine is completed, the second needlepoint 10 side of the needle pipe 3 is disposed within the needle accommodating recess 34a.

Besides, the tube body 33 is provided with a seal member 37 on an outer circumferential surface thereof. The seal member 37 is interposed between the outer circumferential surface of the tube body 33 and the tube hole 2a of the outer tube 2 when the tube body 33 is inserted in the tube hole 2a.

With the seal member 37, an internal space 20 surrounded by the tube hole 2a and a hub main body 11 of the needle hub 4 is sealed in a air-tight manner. In addition, with the seal member 37 put in close contact with the outer circumferential surface of the tube body 33 and the inner wall of the tube hole 2a, the tube body 33 can be stably held in an intermediate portion of the tube hole 2a.

Next, the pusher 36 will be described below.

The pusher 36 includes a plunger 39 composed of a rod-like member, a substantially circular disk-shaped pressing section 40, and the second sealing body 35 which closes the aperture on the other end side of the tube body 33. An axial-directionally one end portion of the plunger 39 is in contact with the second sealing body 35 of the liquid medicine storage section 32. The pressing section 40 is provided at the axial-directionally other end of the plunger 39. The tube body 33, with the liquid medicine M confined in a liquid medicine space 45 surrounded by the first sealing body 34 and the second sealing body 35 of the pusher 36 therein, is stored in the tube hole 2a of the outer tube 2.

Besides, the plunger 39 is provided with a lock section 47 on an outer circumferential surface thereof. The lock section 47 is disposed in the vicinity of the pressing section 40 of the plunger 39. The lock section 47 is formed continuously along the circumferential direction of the outer circumferential surface of the plunger 39. The lock section 47 is a projection projecting in a roughly triangular shape from the outer circumferential surface of the plunger 39.

When the pusher 36 is pushed in along the axial direction of the tube hole 2a of the outer tube 2 to the side of the needle hub 4, as shown in FIG. 4, the lock section 47 is locked by a stopper 17 of the outer tube 2. The lock section 47 and the stopper 17 constitute a holding section 38 by which the plunger 39 and the second sealing body 35 are held at positions reached when they are completely pushed in.

Incidentally, while an example wherein the lock section 47 is formed continuously along the circumferential direction of the outer circumferential surface of the plunger 39 has been described in this embodiment, this is not restrictive. For instance, lock sections 47 may be formed intermittently on the outer circumferential surface of a plunger 39.

In addition, when the pressing section 40 is pressed by the user, as shown in FIG. 4, the liquid medicine storage section 32 is pushed out in the axial direction of the tube hole 2a of the outer tube 2 by the plunger 39 and the second sealing body 35. Then, one end of the tube body 33 of the liquid medicine storage section 32 makes contact with the other end face 11b of the hub main body 11. In this instance, the second needlepoint 10 of the needle pipe 3 punctures the first sealing body 34 of the liquid medicine storage section 32. This results in passage of the liquid into and through the needle pipe 3.

After the second needlepoint 10 is made to puncture the liquid medicine storage section 32 and passage of the liquid into and through the needle pipe 3 is completed, the pressing section 40 is further pressed. This results in that the second sealing body 35 is slid within the tube body 33 of the liquid medicine storage section 32 by the plunger 39, and is pushed out to the side of the first sealing body 34. Thus, with the second sealing body 35 slid within the tube body 3 along the axial direction, the liquid medicine M in the liquid medicine storage section 32 is pushed out by the second sealing body 35, and passes through the second needlepoint 10 of the needle pipe 3, to be discharged through the first needlepoint 10 into the living body.

In this instance, the lock section 47 provided on the plunger 39 is locked by the stopper 17 of the outer tube 2. As a result, the plunger 39 and the second sealing body 35 are held in the outer tube 2 and the tube body 33 of the liquid medicine storage section 32 in the condition where the plunger 39 is completely pushed in. Therefore, even if the force with which the pressing section 40 is pressed is weakened after completion of the administration of the liquid medicine M, the pusher 36 can be prevented from moving toward the side of the other end portion of the outer tube 2.

The other constituents are the same as those of the prefilled syringe 1 according to the first embodiment described above, and, accordingly, description of the other constituents are omitted. According to the prefilled syringe 31 configured as above, also, the same operation and effects as those of the prefilled syringe 1 according to the first embodiment described above can be obtained.

### <3. Third Embodiment>

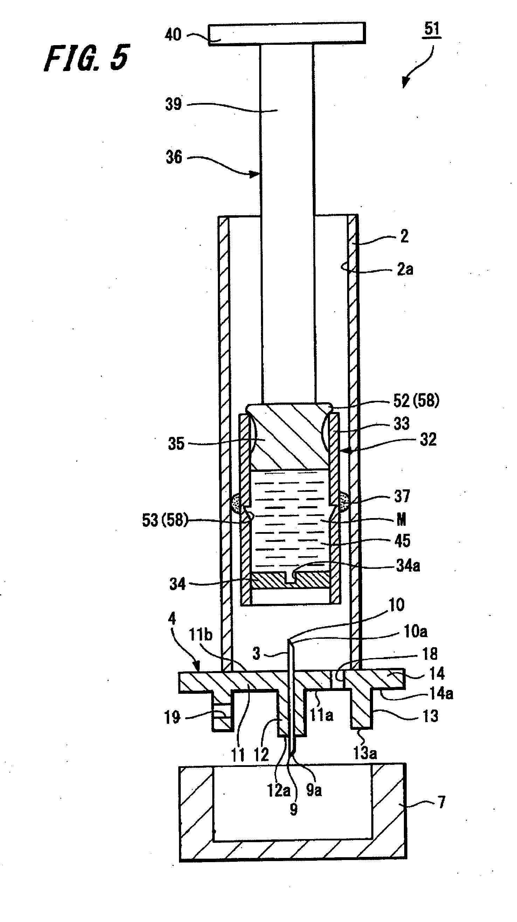

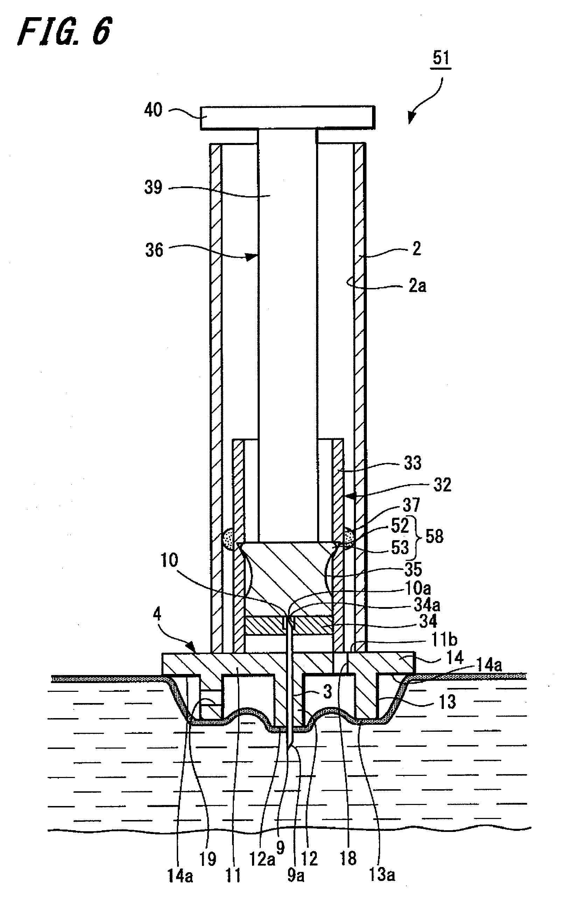

Next, a third embodiment of the prefilled syringe according to the present invention will be described below, referring to FIGS. 5 and 6.

FIGS. 5 and 6 are sectional views showing a prefilled syringe according to this embodiment.

The prefilled syringe 51 according to the third embodiment differs from the prefilled syringe 31 according to the second embodiment in that the configurations of the lock section and the stopper are modified. Here, therefore, a lock section and a stopper will be described, the same parts as those in the prefilled syringe 1 according to the first embodiment and the prefilled syringe 31 in the second embodiment are denoted by the same reference letters or numerals as used above, and overlapping descriptions of the parts will be omitted.

As shown in FIG. 5, a lock section 52 is provided as part of a second sealing body 35 of a pusher 36. The lock section 52 is provided on the side of one end, to be contacted by a plunger 39, of the second sealing body 35. The lock section 52 is a projection projecting in a roughly hemispherical shape from an outer circumferential surface of the second sealing body 35. In addition, the lock section 52 is formed continuously along the circumferential direction of the outer circumferential surface of the second sealing body 35.

A stopper 53 is formed as part of a tube body 33 of a liquid medicine storage section 32. The stopper 53 is a groove section formed at an intermediate portion of the inner wall of the tube body 33. In addition, the stopper 53 is continuous along the circumferential direction of the inner wall of the tube body 33. Besides, the stopper 53 and the lock section 52 constitute a holding section 58.

When a pressing section 40 is pressed and the second sealing body 35 is slid within the tube body 33 along the axial direction, as shown in FIG. 6, the lock section 52 provided as part of the second sealing body 35 is locked by the stopper 53 formed at the inner wall of the tube body 33. This ensures that the second sealing body 35 is held in a position reached when it is completely pushed in within the tube body 33 of the liquid medicine storage section 32. As a result, it is ensured that even if the force with which the pressing section 40 is pressed is weakened, the second sealing body 35 and the pressing section 40 can be prevented from being pushed back by the liquid medicine M, and the pressed state can be maintained.

The other constituents are the same as those of the prefilled syringe 1 according to the first embodiment described above, and, therefore, descriptions of the other constituents are omitted. According to the prefilled syringe 51 configured as above, also, the same operation and effects as those of the prefilled syringe 1 according to the first embodiment described above can be obtained.

### <4. Fourth Embodiment>

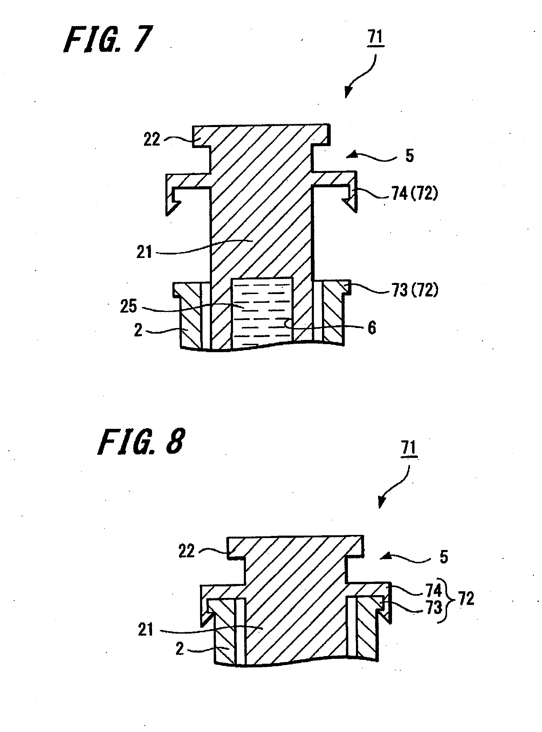

Next, a fourth embodiment of the prefilled syringe according to the present invention will be described below, referring to FIGS. 7 and 8.

FIGS. 7 and 8 are sectional views showing an essential part of a prefilled syringe according to the fourth embodiment.

The prefilled syringe 71 according to the fourth embodiment differs from the prefilled syringe 1 according to the first embodiment in the configuration of a stopper and a lock section which are provided to constitute a holding section. Here, therefore, the lock section and the stopper will be described, the same parts as those in the prefilled syringe according to the first embodiment are denoted by the same reference letters or numerals as used above, and overlapping descriptions of the parts will be omitted.

As shown in FIG. 7, a holding section 72 includes a stopper 73 provided as part of an outer tube 2, and a lock section 74 provided as part of a liquid medicine container 5. The stopper 73 is an outer flange projecting radially outward from an outer circumferential surface of an end portion, on the side opposite to the side where a needle hub 4 is provided, of an outer tube 2. The lock section 74 is provided at a portion, in the vicinity of a pressing section 22, of a container main body 21. The lock section 74 is projecting from the outer circumferential surface of the container main body 21, and is formed in a hook-like sectional shape.

When the pressing section 22 is pressed, as shown in FIG. 8, the lock section 74 of the liquid medicine container 5 is locked by the stopper 73. As a result, it is ensured that even if the force with which the pressing section 22 is pressed is weakened, the liquid medicine container 5 can be prevented from being pushed back by injection resistance of a liquid medicine M, and a pressed state can be maintained.

The other constituents are the same as those of the prefilled syringe 1 according to the first embodiment described above, and, accordingly, descriptions of the other constituents are omitted. According to the prefilled syringe 71 configured as above, also, the same operation and effects as those of the prefilled syringe 1 according to the first embodiment described above can be obtained.

Incidentally, the present invention is not limited to the embodiments described above and shown in the drawings, and various modifications are possible within the scope of the gist of the invention as set forth in the claims. Incidentally, while an example wherein the pressing yardstick section is formed in a flange shape has been described in the embodiments above, this is not restrictive. For instance, a pressing yardstick section can be formed by a method in which the outer circumferential surface of a hollow cylindrical section or the stabilization section may be cut out substantially perpendicularly to provide a stepped section.

In addition, while an example wherein the needle hub is provided with the adjustment section, the stabilization section and the guide section, which is the pressing yardstick section, for administration of a liquid medicine into an upper layer part of skin has been described above, this is not restrictive; for example, the adjustment section, the stabilization section or the guide section may be omitted. In addition, the prefilled syringe according to the present invention is applicable also to a prefilled syringe for puncturing deeper than the upper layer part of skin in administering a liquid medicine. Furthermore, the size of the needle pipe is not limited to 22 to 33 gauge, and may be appropriately selected according to the purpose.

### EXPLANATIONS OF LETTERS OR NUMERALS

1, 31, 51, 71: Prefilled syringe, 2: Outer tube, 2a: Tube hole, 3: Needle pipe (double-ended needle), 4: Needle hub, 5: Liquid medicine container, 6, 32: Liquid medicine storage section, 7: Cap, 8, 38, 58, 72: Holding section, 9: First needlepoint, 10: Second needlepoint, 11: Hub main body, 12: Adjustment section, 12a: Needle protrusion surface, 13: Stabilization section, 13a: End face, 14: Guide section, 14a: Contact surface, 17, 53, 73: Stopper, 18: First vent hole, 19: Second vent hole, 20: Internal space, 21: Container main body, 22, 40: Pressing section, 23: Sealing body, 24, 37: Seal member, 25, 45: Liquid medicine space, 27, 47, 52, 74: Lock section, 33: Tube body, 34: First sealing body, 35: Second sealing body (Sealing body), 36: Pusher, 39: Plunger, B: Bevel length, L: Protrusion length, S: Distance from circumferential edge of needle protrusion surface to circumferential surface of needle pipe, T: Distance from inner wall surface of stabilization section to outer circumferential surface of adjustment section, x: Guide section length, y: Guide section height, d: Inside diameter.

## Claims

1. A prefilled syringe for administering a liquid medicine into a living body, comprising:
a double-ended needle having a needlepoint capable of puncturing the living body;
a needle hub for holding the double-ended needle;
a outer tube which is provided with the needle hub at an end portion thereof and has a tube hole;
a liquid medicine storage section which is disposed in the tube hole of the outer tube and in which the liquid medicine is stored;
a sealing body for sealing the liquid medicine storage section;
a pressing section which operates the liquid medicine storage section so that the liquid medicine storage section can move in an axial direction of the tube hole of the outer tube; and
a holding section which, in a pressed state wherein the liquid medicine storage section and the pressing section have been moved toward the needle hub side until the liquid medicine stored in the liquid medicine storage section has been discharged, holds the pressed state of the pressing section.

2. The prefilled syringe according to claim 1, comprising:
a liquid medicine container which includes the pressing section and the liquid medicine storage section,
wherein the holding section is composed of:
a lock section provided on the liquid medicine container; and
a stopper which is provided on the outer tube and by which the lock section is locked.

3. The prefilled syringe according to claim 1,
wherein the pressing section includes the sealing body which slides within the liquid medicine storage section, and a plunger which connects the pressing section and the sealing body, and
the holding section is composed of:
a lock section provided on the plunger; and
a stopper which is provided on the outer tube and by which the lock section is locked.

4. The prefilled syringe according to claim 1,
wherein the pressing section is provided with the sealing body which slides within the liquid medicine storage section, and
the holding section is composed of:
a lock section provided on the sealing body; and
a stopper which is provided in the liquid medicine storage section and by which the lock section is locked.

5. The prefilled syringe according to claim 1,
wherein a holding force with which the pressing section is held by the holding section is set to be not less than 3 N.

6. The prefilled syringe according to claim 1,
wherein an adjustment section having a needle protrusion surface from which the needlepoint of the double-ended needle protrudes is provided in a periphery of the double-ended needle.

7. The prefilled syringe according to claim 1, comprising:
a stabilization section disposed so as to cover a periphery of the needlepoint of the double-ended needle, the stabilization section having an end face which makes contact with a skin when the double-ended needle is made to puncture the living body.
